(19) **Europäisches Patentamt / European Patent Office / Office européen des brevets**

(11) **EP 2 238 893 A1**

(12) # EUROPEAN PATENT APPLICATION

(43) Date of publication:
**13.10.2010 Bulletin 2010/41**

(51) Int Cl.:
**A61B 5/00** (2006.01)

(21) Application number: **09176865.5**

(22) Date of filing: **24.11.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **06.04.2009 JP 2009092197**

(71) Applicant: **Sumitomo Electric Industries, Ltd.**
**Osaka-shi, Osaka 541-0041 (JP)**

(72) Inventor: **Nakaji, Haruo**
**Kanagawa (JP)**

(74) Representative: **Spilgies, Jan-Hendrik**
**Hoffmann Eitle**
**Patent- und Rechtsanwälte**
**Arabellastrasse 4**
**81925 München (DE)**

(54) **Dental diagnostic system by means of optical coherence tomography**

(57)    A dental diagnostic OCT system capable of diagnosis of dental caries existing at the approximal surfaces of teeth, comprising a light source (10), a beam splitter (20), a mirror (62), a photo detector (40), a lens (51), an optical fiber (52), and a probe (60). A filler having a refractive index larger than the refractive index of air and smaller than that of an object of diagnosis is to be filled between the tip portion of the probe and approximal surfaces of teeth, i.e., objects of diagnosis. The light output from the tip portion of the probe is irradiated to the objects of diagnosis via the filler, and the sample light occurring upon such irradiation is input into the tip portion of the probe via the filler. The intensity of light which the beam splitter outputs by combining the sample light and the reference light is detected by the photo detector, and thereby the teeth as the objects of diagnosis are diagnosed.

FIG. 1

1

EP 2 238 893 A1

**Description**

BACKGROUND OF THE INVENTION

Field of the Invention

The present invention relates to a dental diagnostic system by means of optical coherence tomography.

Description of the Background Art

Optical coherence tomography (OCT) is a technique for detecting light reflected or scattered at a position specified by position resolution on the order of coherent length with respect to the direction of travel of light, and displaying the intensity distribution of the reflected or scattered light by tomographic images. OCT is used for, for example, eyeball diagnosis or dental diagnosis. Techniques in which the OCT is used for dental diagnosis are described in Japanese Patent Application Publication No. 2007-225321 and "Real-time in vivo imaging of dental tissue by means of optical coherence tomography (OCT)" by R. Brandenburg, B. Haller, and C. Hauger, Optics Communications 227(2003) 203-211.
If early dental caries can be discovered, it will be possible to achieve a "non-scraping treatment of dental caries" by application of fluoride or the like, for example. The predilection sites of dental caries are occlusal surfaces (clenching parts of teeth), smooth surfaces (between teeth and gingiva, periodontal pockets), approximal surfaces (between a tooth and a tooth). Of these, particularly the caries of approximal dental surfaces are difficult to detect by either ocular inspection or X-ray diagnosis, and the establishment of technique for early diagnosis is desired. In such situation, the OCT system for dental diagnostic use has attracted much attention as one of prospective system for enabling early diagnosis of caries occurring in approximal dental surfaces.

SUMMARY OF THE INVENTION

An object of the present invention is to provide an optical coherence tomography system for dental diagnosis (hereinafter, referred to as "dental diagnostic OCT system" with which the diagnosis of dental caries in approximal surfaces can be done.
To achieve the object, provided is a dental diagnostic OCT system which comprises (1) a light source for emitting light, (2) a separating part for outputting first split light and second split light by separating the light emitted from the light source, (3) a probe which radiates the first split light to a tooth, i.e., an object of diagnosis, and which receives and guides as sample light the light reflected or scattered at the surface or inner part of the object of diagnosis upon such irradiation, and which includes a tip portion for outputting the first split light, the tip portion being arranged at a position opposed to the object of diagnosis through a filler to be filled therebetween, the refractive index of the filler being larger than the refractive index of air and smaller than the refractive index of the object of diagnosis, (4) a combining part for receiving the sample light that has been guided by the probe and has reached there, the combining part also receiving as reference light the second split light that has been output from the separating part and has reached there, so that the sample light and the reference light are combined therein, and (5) a photo detector for detecting the intensity of light output from the combining part.
This dental diagnostic OCT system may have a light source capable of outputting broadband light and further may have an optical path length operation means capable of altering the optical path length between the separating part and the combining part with respect to the second split light and the reference light. Or, in this dental diagnostic OCT system, the light source may be a light source outputting narrowband light and capable of altering the central wavelength. (The former is a system using time domain OCT, and the latter is a system using Fourier domain OCT.)
Also, in this dental diagnostic OCT system, the tip portion of the probe may have a wedge form and the filler may be filled between the tip portion and the object of diagnosis. In this case, the wedge-shaped tip portion of the probe is preferably made of silicone rubber that is transparent to light emitted by the light source.
In addition, in this dental diagnostic OCT system, the filler may be glycerol or aqueous solution. Preferably, the probe irradiates the first split light of p-polarized light to the object of diagnosis.
It is possible to adopt an arbitrary combination with respect to the type of the interferometer regarding the time domain OCT or Fourier domain OCT, the shape and material of the probe, the kind of the filler, and the polarization state of the first split light.
A method of dental diagnosis is provided as another aspect of the present invention. This method of dental diagnosis uses a dental diagnostic OCT system which comprises: a light source; a separating part for outputting first split light and second split light by separating the light emitted from the light source into two; a probe which radiates the first split light to a tooth, i.e., an object of diagnosis, and which receives as sample light the light reflected or scattered at the surface or inner part of the object of diagnosis upon such irradiation; a combining part for combining the sample light and the second split light; and a photo detector for detecting the intensity of the combined light, and the method of dental diagnosis comprises: (1) a step of positioning the tip portion of the probe at a position opposed to the object of diagnosis in a manner such that a filler is to be arranged between the tip portion and the object of diagnosis, the filler having a refractive index that is larger than the refractive index of air and smaller than the refractive index of the object of diagnosis, (2) a step of irradiating the first split light from the probe tip to an object of diagnosis through the filler, and receiving as sample light through the filler, the light reflected or scattered at the surface or inner part of the object of diagnosis upon such irradiation, (3) a step of causing interference between the sample light and the reference light that is the second split light, and (4) a step of seeking tomographic images of the object of diagnosis based on the intensity of the light thus interfered.

BRIEF DESCRIPTION OF THE DRAWINGS

Figure 1 is a conceptional schematic diagram showing a dental diagnostic OCT system according to a first embodiment of the present invention.

Figure 2 is an enlarged partial view showing the probe contained in the dental diagnostic OCT system of Fig. 1.

Figure 3 is a photograph of the buccal side (buccal face) of the jowl of a pig which is the object of diagnosis used in the example.

Figure 4 is tomographic images obtained by scanning with the light along the three lines X, Y, Z shown in Fig. 3.

Figure 5 is a conceptional schematic diagram showing a dental diagnostic OCT system according to a second embodiment of the present invention.

Figure 6 is an enlarged partial view showing the probe contained in the dental diagnostic OCT system of the present invention.

DETAILED DESCRIPTION OF THE INVENTION

The above-mentioned features and other features, aspects, and advantages of the present invention will be better understood through the following description, appended claims, and accompanying drawings. In the explanation of the drawings, an identical mark is applied to identical elements and an overlapping explanation will be omitted.

The reflection of light at the interface between two media having mutually different refractive indexes is shown by Fresnel equations (1a) and (1b):

$$r_p = \frac{n_2^2 \cos\phi_0 - n_1 \sqrt{n_2^2 - n_1^2 \sin^2\phi_0}}{n_2^2 \cos\phi_0 + n_1 \sqrt{n_2^2 - n_1^2 \sin^2\phi_0}} \qquad (1a),$$

$$r_s = \frac{n_1 \cos\phi_0 - \sqrt{n_2^2 - n_1^2 \sin^2\phi_0}}{n_1 \cos\phi_0 + \sqrt{n_2^2 - n_1^2 \sin^2\phi_0}} \qquad (1b),$$

where, $r_p$ is an amplitude reflectivity of p-polarized light, and $r_s$ is an amplitude reflectivity of s-polarized light. Also, $n_1$ is a refractive index of a first medium, $n_2$ is a refractive index of a second medium, and $\phi_0$ is an incident angle. For example, the first medium is air, and the second medium is enamel. The refractive index of enamel is about 1.6.

The reflectivity $R_p$ of p-polarized light and the reflectivity $R_s$ of s-polarized light are shown by (2a) and (2b), respectively:

$$R_p = r_p r_p^* \qquad (2a),$$

$$R_s = r_s r_s^* \qquad (2b),$$

where, $r_p^*$ is a complex conjugate of $r_p$, and $r_s^*$ is a complex conjugate of $r_s$. As can be seen from these formulas, the reflectivity of both s-polarized light and p-polarized light increases rapidly when the incident angle $\phi_0$ approaches 90 degrees.

When a tomographic image of approximal surface of a tooth is obtained by the optical coherence tomography (OCT) system for dental diagnosis, the light is generally irradiated from the buccolingual direction to the approximal surface.

When light is irradiated from the buccolingual direction, the light cannot easily enter into the enamel because Fresnel's reflection at the surface of the enamel is large since the surface of the tooth has curvature. Also, if the surface of the tooth is uneven, a diffuse reflection occurs there, which makes it difficult for the light to enter into the enamel. And, when the light that enters into the enamel is little, the measurable depth becomes shallow, and accordingly it is impossible to accomplish diagnosis of dental caries at the approximal surface of the tooth. Thus, the present inventor found that when the diagnosis of dental caries in the approximal surface of tooth is done with a conventional OCT system, a troublesome problem is the Fresnel reflection of light due to differences in the refractive indexes at the interface between air and teeth (enamel).

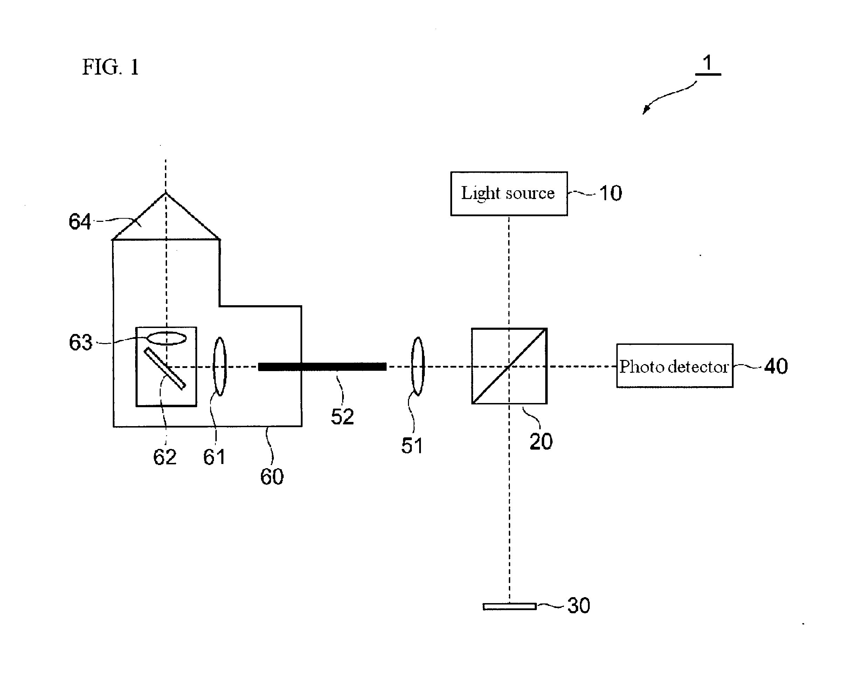

Figure 1 is a conceptional schematic diagram showing a dental diagnostic OCT system 1, which is a first embodiment of the present invention. The dental diagnostic OCT system 1 includes time domain optical coherence tomography, and is equipped with a light source. 10, a beam splitter 20, a mirror 30, a photo detector 40, a lens 51, an optical fiber 52, and a probe 60.

The light source 10 outputs low-coherence light, and hence a superluminescent diode is preferable for the light source, for example. The beam splitter 20 is used as a separating part which outputs first split light and second split light by separating the light output from the light source 10. The beam splitter 20 outputs the first split light to the lens 51 and the second split light to the mirror 30.

The mirror 30 receives the second split light output from the beam splitter 20 and reflects the light to the beam splitter 20. The lens 51 receives the first split light output from the beam splitter 20, and focuses the light on the end face of the optical fiber 52 so as to waveguide the light through the optical fiber 52. Also, the lens 51 collimates the light diffusely output from the end face of the optical fiber 52 and inputs the light into the beam splitter 20.

The beam splitter 20 receives the light (reference light) reflected by the mirror 30 and reached there, as well as the light (sample light) which has been output from the end face of the optical fiber 52 and collimated by the lens 51. Then, it combines the reference light and the sample light and inputs the combined light to the photo detector 40. That is, the beam splitter 20 is used not only as the separating part, but also as the combining part for combining the reference light and the sample light.

The photo detector 40 detects the intensity of light output from the beam splitter 20. Here, it is preferable that an optical fiber for guiding light be provided in the optical system between the light source 10 and the beam splitter 20, the optical system between the beam splitter 20 and the mirror 30, and the optical system between the beam splitter 20 and the photo detector 40, respectively. In such case, it is preferable to provide a lens for focusing light so as to make incident on an end face of the optical fiber, as well as a lens for collimating the light output from an end face of the optical fiber.

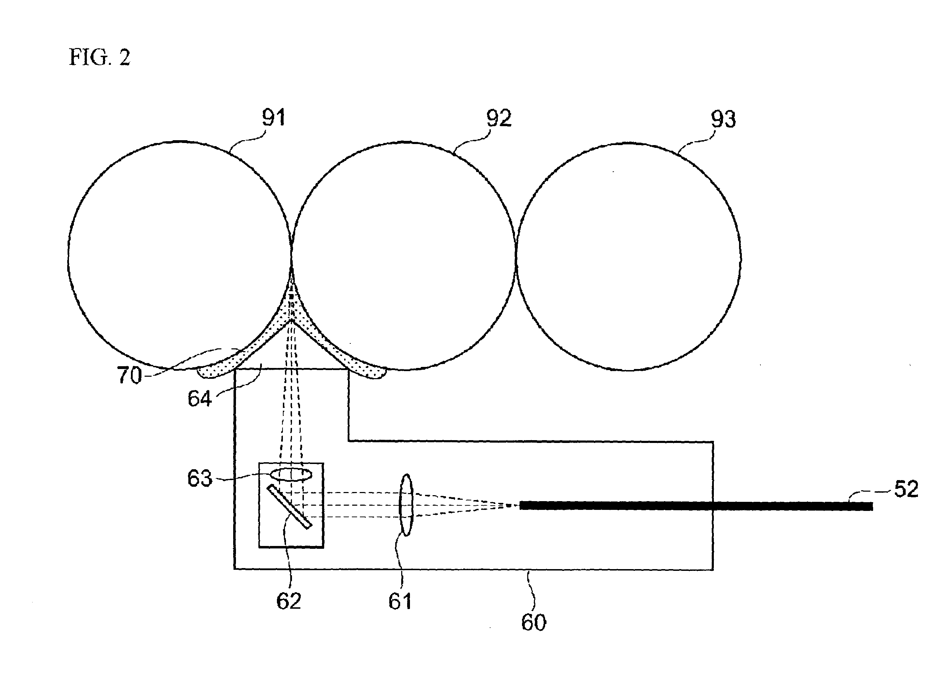

Figure 2 is an enlarged partial view showing the probe 60 contained in the dental diagnostic OCT system of Fig. 1, The probe 60 integrally has a lens 61, a mirror 62, a lens 63, and a tip portion 64 and is connected with the optical fiber 52.

In Fig. 2, teeth are shown as objects of diagnosis 91 to 93.

The lens 61 collimates the light diffusely output from the end face of the optical fiber 52. The mirror 62 reflects the light collimated by the lens 61 to the lens 63. The light which has been reflected by the mirror 62 and reached there is converged by the lens 63. The tip portion 64 has a wedge-like shape having a flat bottom face, and preferably is made of silicone rubber. A filler 70 is filled between the tip portion 64 and the approximal surfaces of the objects of diagnosis 91 to 93. The refractive index of the filler 70 is larger than the refractive index of air and is smaller than the refractive index of the enamel of the objects of diagnosis 91 to 93.

In the probe 60, the light output from the end face of the fiber 52 is collimated by the lens 61, is reflected by the mirror 62, is converged by the lens 63, and is irradiated to the approximal surfaces of objects of diagnosis 91 to 93 via the tip portion 64 and the filler 70. In such case, if p-polarized light is irradiated through the probe 60 to the objects of diagnosis, it would be preferable because the reflectivity at the surface of the objects of diagnosis 91 to 93 is small.

Also, upon such irradiation, the light (sample light) reflected or scattered at the surface or inside of the objects of diagnosis 91 to 93 is collimated by the lens 63 via the filler 70 and the tip portion 64 so as to be reflected by the mirror 62, and is focused by the lens 63 to the end face of the optical fiber 52. The sample light focused to the end face of the optical fiber 52 is waveguided by the optical fiber 52, and is input to the photo detector 40 via the lens 51 and the beam splitter 20.

As described above, the dental diagnostic OCT system 1 is used in a manner such that the filler 70 having a refractive index that is larger than the refractive index of air and smaller than the refractive index of the object of diagnosis is filled between the tip portion 64 of the probe 60 and the approximal surfaces of the object of diagnosis 91 to 94. The light output from the tip portion 64 of the probe 60 is irradiated to the objects of diagnosis via the filler 70, and the sample light arising upon such irradiation is input into the tip portion 64 of the probe 60 via the filler 70. The light intensity of the sample light and reference light which are combined and output by the beam splitter 20 is detected by the photo detector 40, and thereby the teeth as the objects of diagnosis are diagnosed.

In such case, the mirror 62 and the lens 63 are integrally moved in the direction parallel to the optical axis of the lens 61. Also, an optical path length operation means is provided for moving the mirror 30 so as to change the optical path length between the mirror 30 and the beam splitter 20 shown in Fig. 1. By the above-described scanning operation, 3-D tomographic images of objects of diagnosis 91 to 93 are obtained.

In the dental diagnostic OCT system 1, the filler 70 is to be filled between the tip portion 64 of the probe 60 and the approximal surfaces of objects of diagnosis 91 to 94, and the refractive index of the filler 70 is larger than the refractive index of air and smaller than the refractive index of the objects of diagnosis. By the use of the filler 70, the Fresnel reflection due to the curvature of teeth and the diffuse reflection due to the unevenness of the surface of teeth are restrained, allowing more light to penetrate into the enamel of teeth, and accordingly the measurable depth becomes deeper. Thus, it is made possible to accomplish the diagnosis of dental caries in the adjacent parts between the teeth.

The refractive index of the filler 70 is preferably close to 1.6, which is the refractive index of the enamel of teeth, i.e., objects of diagnosis. In order to restrain the surface reflection, water having a refractive index of 1.33 can also be used as the filler 70. However, greater surface reflection restraining effect could be obtained by using glycerol as the filler 70 since the refractive index of the glycerol is 1.46, which is closer to the refractive index of the enamel than water.

Since the viscosity of the glycerol is greater than the viscosity of water, however, poor wetting of the teeth surface would occur. As a result, a bubble might easily be generated between the glycerol and the teeth surface, and the reduction of the surface reflection could not be expected very much. Therefore, it is preferable to consider not only the refractive index but also the viscosity which affects wettability with respect to the filler 70.

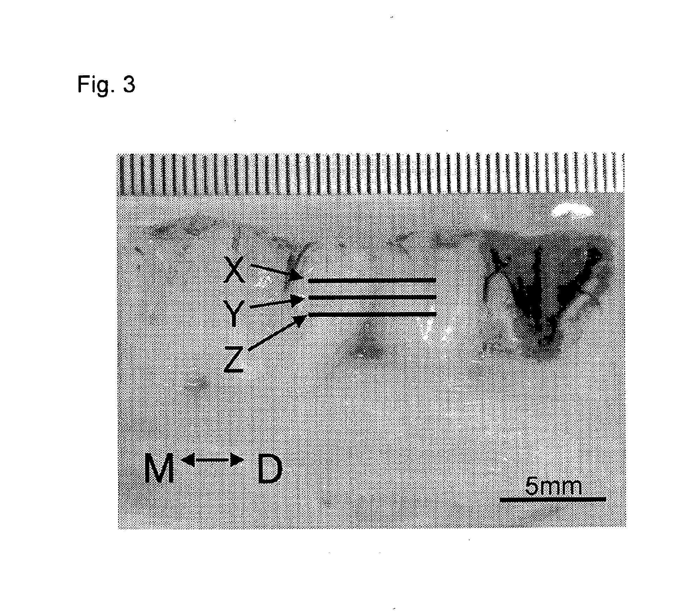

Next, an example in which the dental diagnostic OCT system 1 is used will be described. Figure 3 is a photograph of the buccal side (buccal face) of the jowl of a pig which is the object of diagnosis used in the example. In the example, the light was irradiated in a direction perpendicular to the buccal face (i.e., in Fig. 3, perpendicular to the surface of the page). The conditions of the example are the same as the conditions specified in Brandenburg. However, the measurement was done ex vivo (in vitro) in this example, whereas in Brandenburg, the measurement is done in vivo.

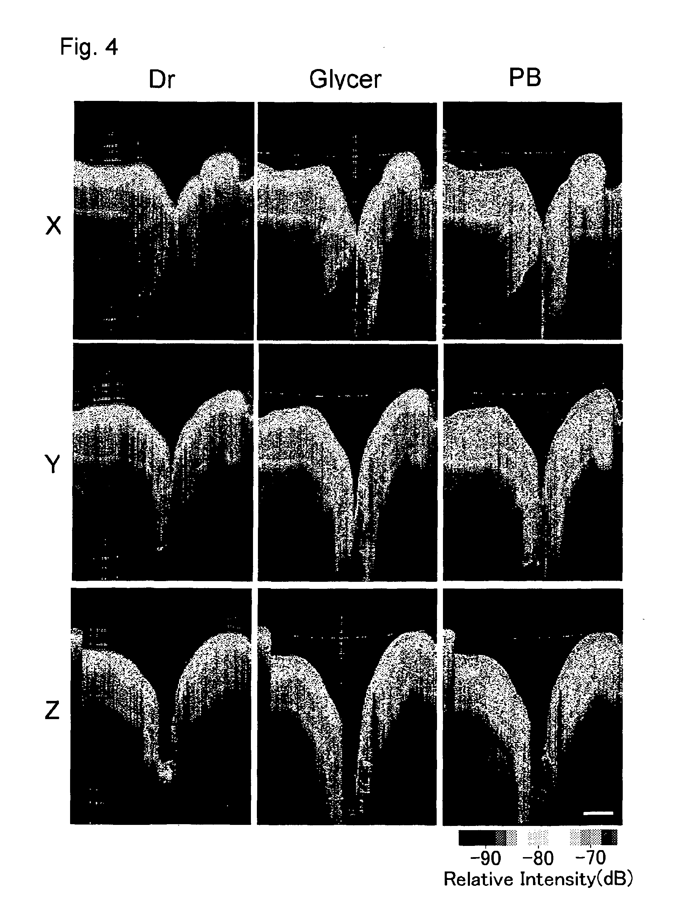

Figure 4 is tomographic images obtained by scanning with the light along the three lines X, Y, Z shown in Fig. 3: the row X of Fig. 4 shows tomographic images obtained when the scanning of light was done along line X; the row Y shows tomographic images obtained when the scanning of light was done along the line Y; and the row Z shows tomographic images obtained when the scanning of light was done along the line Z. The column Dr shows tomographic images obtained in the case where the filler 70 was not used. The column Glycerol shows tomographic images obtained when glycerol was used as the filler 70. The column PB shows tomographic images obtained when PBS (phosphate buffered saline) was used as the filler 70.

These tomographic images are formed in a gray scale according to the intensity of back-scattering light: the less dark parts show that more light was back-scattered therefrom; and in contrast, the darker parts show that the light was not much scattered therefrom.

As can been seen by comparing these tomographic images, more reflection or scattering occurs at the surface and accordingly less light penetrates into the inside in the case (the row Dr) where the filler 70 is not used. On the other hand, when glycerol or PBS is used as the filler 70, both the Fresnel reflection due to the curvature of teeth and the diffuse reflection (reflection or scattering of light at the surfaces of teeth) due to the unevenness of the surface of teeth occur less, and accordingly more light penetrates into the enamel. Consequently, by using the dental diagnostic OCT system 1, the diagnosis of dental caries in the adjacent parts between teeth can be accomplished since the measurable depth becomes deeper.

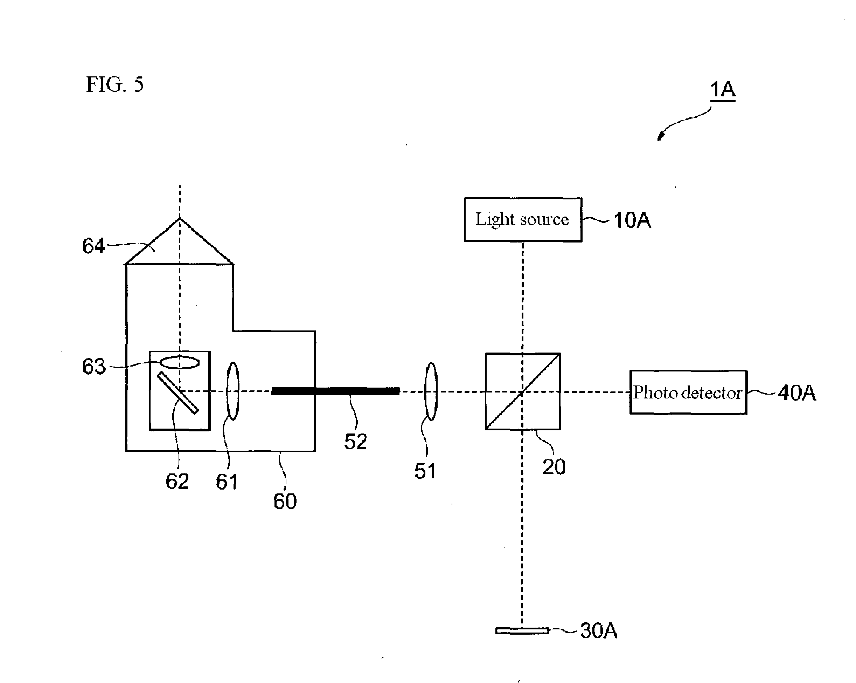

Figure 5 is a conceptional schematic diagram showing a dental diagnostic OCT system 1 A according to a second embodiment of the present invention. The dental diagnostic OCT system 1A includes Fourier domain optical coherence tomography and is equipped with a light sources 10A, a beam splitter 20, a mirror 30A, a photo detector 40A, a lens 51, an optical fiber 52, and a probe 60.

The light source 10A outputs narrowband light and the central wavelength can be changed. The mirror 30A receives the second split light output from the beam splitter 20, and reflects the light to the beam splitter 20. The optical path length between the mirror 30A and the beam splitter 20 is constant. The photo detector 40A detects the intensity of light output from the beam splitter 20, and seeks the distribution of the intensity vs. the depth by conducting Fourier transform of the intensity distribution for each frequency component. The other elements are identical with those of the dental diagnostic OCT system 1.

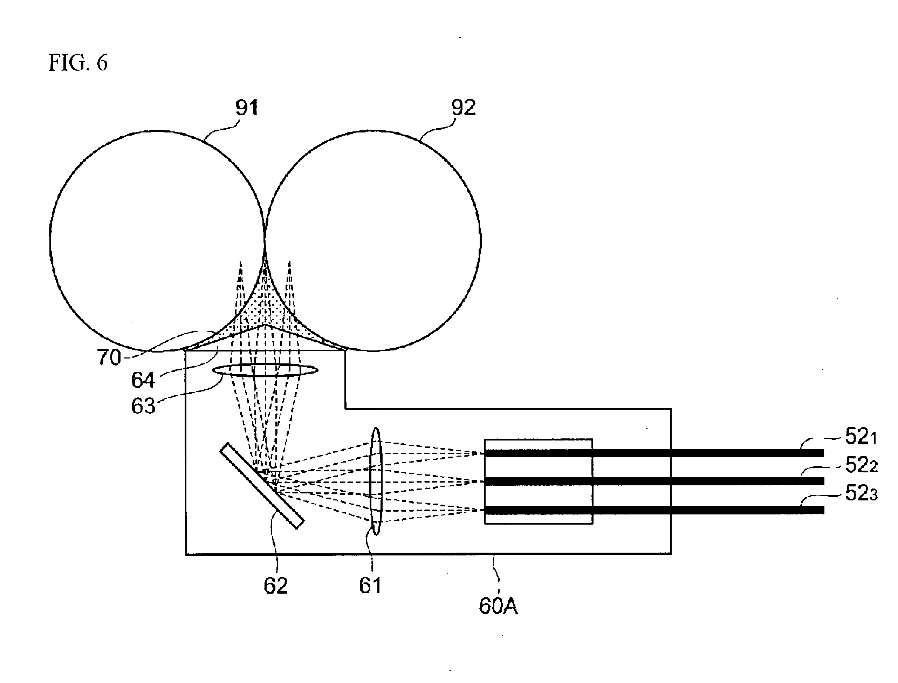

Figure 6 shows the structure of a modified example of the probe 60 included in the dental diagnostic OCT system 1 or the dental diagnostic OCT system I A. The probe 60A of the modified example is the same as the above-mentioned probe 60 in that it comprises a lens 61, a mirror 62, a lens 63, and a tip portion 64 in an integral form, but it differs from the probe 60 in that it is connected with a plurality of optical fibers 52. Also, the probe 60A is different from the probe 60 in that it is unnecessary to move the mirror 62 and lens 63. With the probe 60A, the reflection or scattering at the same number of positions as the number of the optical fibers 52 can be detected at the same time, and accordingly the diagnosis of dental caries can be performed in a short time.

While this invention has been described in connection with what is presently considered to be the most practical and preferred embodiments, the invention is not limited to the disclosed embodiments, but on the contrary, is intended to cover various modifications and equivalent arrangements included within the and scope of the appended claims.

**Claims**

1. A dental diagnostic OCT system comprising:

   a light source for emitting light;
   a separating part for outputting first split light and second split light by separating the light emitted from the light source;
   a probe for irradiating the first split light to a tooth, i.e., an object of diagnosis and receiving and guiding as sample light the light reflected or scattered at the surface or inner part of the object of diagnosis upon such irradiation, the probe including a tip portion for outputting the first split light, the tip portion being arranged at a position opposed to the object of diagnosis through a filler to be arranged therebetween, the refractive index of the filler being larger than the refractive index of air and smaller than the refractive index of the object of diagnosis;
   a combining part for receiving the sample light that has been guided by the probe and has reached there, the combining part also receiving as reference light the second split light that has been output from the separating part and has reached there, so that the sample light and the reference light are combined therein; and
   a photo detector for detecting the intensity of light output from the combining part.

2. The dental diagnostic OCT system according to claim 1, wherein the light source is a light source for outputting broadband light, and the dental diagnostic OCT system further comprises an optical path length operation means capable of altering the optical path length between the separating part and the combining part with respect to the second split light and the reference light.

3. The dental diagnostic OCT system according to claim 1, wherein the light source outputs narrowband light and the central wavelength can be changed.

4. The dental diagnostic OCT system according to any one of the preceding claims, wherein the tip portion of the probe has a wedge form and the filler is to be filled between the tip portion and the object of diagnosis.

5. The dental diagnostic OCT system according to claim 4, wherein the wedge-shaped tip portion of the probe is made of silicone rubber that is transparent to light emitted by the light source.

6. The dental diagnostic OCT system according to any one of the preceding claims, wherein the filler is glycerol.

7. The dental diagnostic OCT system according to any one of claims 1 to 5, wherein the filler is an aqueous solution.

8. The dental diagnostic OCT system according to any one of the preceding claims, wherein the probe irradiates the first split light of p-polarized light to the object of diagnosis.

9. A method of dental diagnosis, the method using dental diagnostic OCT system comprising: a light source; a separating part for outputting first split light and second split light by separating the light emitted from the light source into two; a probe for irradiating the first split light to a tooth, i.e., an object of diagnosis, and receiving as sample light the light reflected or scattered at the surface or inner part of the object of diagnosis upon such irradiation; a combining part for combining the sample light and the second split light; and a photo detector for detecting the intensity of the combined light, and the method comprising:

   a step of positioning the tip portion of the probe at a position opposed to the object of diagnosis in a manner such that a filler is to be arranged between the tip portion and the object of diagnosis, the filler having a refractive index that is larger than the refractive index of air and smaller than the refractive index of the object of diagnosis;
   a step of irradiating the first split light from the probe tip to an object of diagnosis through the filler, and receiving as sample light through the filler, the light reflected or scattered at the surface or inner part of the object of diagnosis upon such irradiation;
a step of causing interference between the sample light and the reference light, i.e., the second split light; and
a step of seeking tomographic images of the object of diagnosis based on the intensity of the light thus interfered.

**10.** The method of dental diagnosis according to claim 9, wherein the light source outputs broadband light, and the method further comprises a step of altering the optical path length of the second split light.

**11.** The method of dental diagnosis according to claim 9, wherein the light source outputs narrowband light, and the method further comprises a step of altering the central wavelength of the narrowband light.

**12.** The method of dental diagnosis according to any one of claims 9 to 11, wherein the tip portion of the probe has a wedge form.

**13.** The method of dental diagnosis according to claim 12, wherein the wedge-shaped tip portion of the probe is made of silicone rubber that is transparent to light emitted by the light source.

**14.** The method of dental diagnosis according to any one of claims 9 to 13, wherein the filler is glycerol.

**15.** The method of dental diagnosis according to any one of claims 9 to 13, wherein the filler is an aqueous solution.

**16.** The method of dental diagnosis according to any one of claims 9 to 15, wherein the first split light irradiated to the object of diagnosis by the probe is p-polarized light.

FIG. 1

FIG. 2

EP 2 238 893 A1

## Fig. 3

Fig. 4

EP 2 238 893 A1

FIG. 5

1A

Light source 10A

Photo detector 40A

20

30A

51

52

60

61

62

63

64

FIG. 6

EP 2 238 893 A1

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/062429 A1 (LIANG RONGGUANG [US] ET AL) 13 March 2008 (2008-03-13) | 1-3, 8-11,16 | INV. A61B5/00 |
| Y | * paragraph [0002] * <br> * paragraphs [0112] - [0115] * <br> * paragraphs [0125] - [0132] * <br> * paragraphs [0135] - [0138] * <br> * paragraph [0168] * <br> * figures 13A,13B,15B,16,18,24,25 * | 5-7, 12-15 | |
| X | US 2005/283058 A1 (CHOO-SMITH LIN-P ING [CA] ET AL) 22 December 2005 (2005-12-22) | 1-4,8 | |
| Y | * paragraph [0056] * <br> * paragraphs [0173] - [0178] * <br> * paragraph [0182] * <br> * figures 17-25 * | 12 | |
| Y | DE 297 04 274 U1 (REBOLL HANS PETER [DE]) 5 June 1997 (1997-06-05) <br> * page 2, paragraphs 3,4 * <br> * figure 1 * | 5,13 | |
| | | | TECHNICAL FIELDS SEARCHED (IPC) |
| Y | US 2006/200017 A1 (MONFRE STEPHEN L [US] ET AL MONFRE STEPHEN L [US] ET AL) 7 September 2006 (2006-09-07) <br> * paragraphs [0035] - [0039] * <br> * paragraph [0036] * <br> * paragraph [0158] * | 6,7,14, 15 | A61B |

The present search report has been drawn up for all claims

| Place of search | Date of completion of the search | Examiner |
|---|---|---|
| Berlin | 1 July 2010 | Völlinger, Martin |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 09 17 6865

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2010

| Patent document cited in search report | Publication date | Patent family member(s) | Publication date |
|---|---|---|---|
| US 2008062429 A1 | 13-03-2008 | EP 2061371 A2<br>WO 2008033217 A2 | 27-05-2009<br>20-03-2008 |
| US 2005283058 A1 | 22-12-2005 | NONE | |
| DE 29704274 U1 | 05-06-1997 | NONE | |
| US 2006200017 A1 | 07-09-2006 | EP 1850736 A2<br>JP 2008531133 T<br>WO 2006093816 A2 | 07-11-2007<br>14-08-2008<br>08-09-2006 |

EPO FORM P0459

For more details about this annex : see Official Journal of the European Patent Office, No. 12/82

**EP 2 238 893 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007225321 A

**Non-patent literature cited in the description**

- **R. Brandenburg ; B. Haller ; C. Hauger.** Real-time in vivo imaging of dental tissue by means of optical coherence tomography (OCT). *Optics Communications,* 2003, vol. 227, 203-211